(19) Europäisches Patentamt
European Patent Office
Office européen des brevets

(11) Veröffentlichungsnummer: **0 057 384**
**B1**

(12) # EUROPÄISCHE PATENTSCHRIFT

(45) Veröffentlichungstag der Patentschrift: **25.07.84**

(21) Anmeldenummer: **82100404.1**

(22) Anmeldetag: **21.01.82**

(51) Int. Cl.³: **C 07 C121/75**, C 07 C 69/743, C 07 C 69/74, C 07 C 67/14, C 07 C 47/575, C 07 C 43/295, C 07 C 69/612, C 07 C 69/65, A 01 N 53/00

(54) **4-Fluor-3-halophenoxy-benzylester, Verfahren zu deren Herstellung und deren Verwendung in Schädlingsbekämpfungsmitteln sowie neue Zwischenprodukte und Verfahren zu deren Herstellung.**

(30) Priorität: **31.01.81 DE 3103325**

(43) Veröffentlichungstag der Anmeldung: **11.08.82 Patentblatt 82/32**

(45) Bekanntmachung des Hinweises auf die Patenterteilung: **25.07.84 Patentblatt 84/30**

(84) Benannte Vertragsstaaten: **BE CH DE FR GB IT LI NL**

(56) Entgegenhaltungen:
**EP-A- 0 044 139**
**DE-A- 2 615 435**
**DE-A- 2 739 854**
**DE-A- 3 012 302**
**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Fuchs, Rainer, Dr.**
**Roeberstrasse 8**
**D-5600 Wuppertal 1 (DE)**
Erfinder: **Blank, Heinz Ulrich, Dr.**
**Am Geusfelde 35**
**D-5068 Odenthal (DE)**
Erfinder: **Rottloff, Günther**
**Semmelweissstrasse 70**
**D-5000 Köln 1 (DE)**
Erfinder: **Hammann, Ingeborg, Dr.**
**Belfortstrasse 9**
**D-5000 Köln (DE)**
Erfinder: **Homeyer, Bernhard, Dr.**
**Obere Strasse 28**
**D-5090 Leverkusen 3 (DE)**
Erfinder: **Behrenz, Wolfgang, Dr.**
**Untergruendemich 14**
**D-5063 Overath (DE)**
Erfinder: **Stendel, Wilhelm, Dr.**
**In den Birken 55**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 057 384 B1

Jouve, 18, rue St-Denis, 75001 Paris, France

**Beschreibung**

Die Erfindung betrifft neue 4-Fluor-3-halophenoxybenzylester, ein Verfahren zu ihrer Herstellung, ihre Verwendung in Schädlingsbekämpfungsmitteln, sowie neue Zwischenprodukte dafür und Verfahren zu deren Herstellung.

Es ist bekannt, daß bestimmte Phenoxybenzylester, wie z. B. 3-(2,2-Dichlor-vinyl)-2,2-dimethylcyclopropancarbonsäure-3-phenoxybenzylester (Permethrin) und 3-(2,2-Dichlorvinyl)-2,2-dimethylcyclopropancarbonsäure-α-cyano-3-phenoxy-benzylester (Cypermethrin) insektizide und akarizide Eigenschaften aufweisen (vergleiche GB-PS 1 413 491).

Halogensubstituierte Phenoxybenzylester von Cyclopropancarbonsäuren und α-Phenyl-α-isopropylessigsäuren sind bekannt aus DE-OS 26 15 435 und DE-OS 27 39 854.

Die Wirkung dieser Verbindungen ist jedoch, insbesondere bei niedrigen Wirkstoffkonzentrationen und Aufwandmengen, nicht immer zufriedenstellend.

Gegenstand der vorliegenden Erfindung sind

1. Neue 4-Fluor-3-halophenoxy-benzylester der Formel (I)

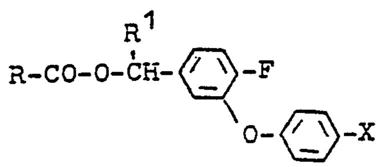

(I)

in welcher R für 2,2,3,3-Tetramethylcyclopropyl oder für den Rest

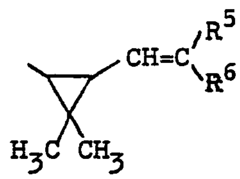

steht, worin

$R^5$ für Wasserstoff, Fluor, Chlor, Brom oder für gegebenenfalls halogen-substituiertes $C_1$-$C_4$-Alkyl steht und

$R^6$ für Fluor, Chlor, Brom, gegebenenfalls halogen-substituiertes $C_1$-$C_4$-Alkyl oder für gegebenenfalls durch Halogen und/oder durch gegebenefalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht oder worin die beiden Reste $R^6$ und $R^5$ zusammen für $C_2$-$C_5$-Alkandiyl (Alkylen) stehen ;

in welcher weiter R für den Rest

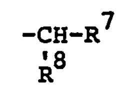

steht, worin

$R^7$ für gegebenenfalls durch Halogen und/oder durch gegebenenfalls halogen-substituierte Reste aus der Reihe $C_1$-$C_4$-alkyl, $C_1$-$C_4$-Alkoxy, $C_1$-$C_4$-alkylthio oder $C_1$-$C_2$-Alkylendioxy substituiertes Phenyl steht und

$R^8$ für Isopropyl oder Cyclopropyl steht ;

$R^1$ für Wasserstoff oder Cyano steht,

X für Chlor oder brom steht ;

2. ein Verfahren zur Herstellung der 4-Fluor-3-halophenoxybenzylester der Formel I

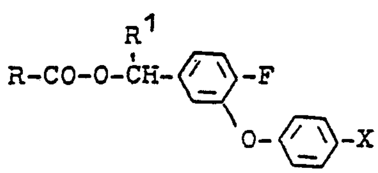

(I)

in welcher

R für 2,2,3,3-Tetramethylcyclopropyl oder für den Rest

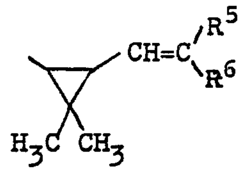

steht, worin

$R^5$ für Wasserstoff, Fluor, Chlor, Brom oder für gegebenenfalls halogen-substituiertes $C_1$-$C_4$-Alkyl steht und

$R^6$ für Fluor, Chlor, Brom, gegebenenfalls halogensusbstituiertes $C_1$-$C_4$-Alkyl oder für gegebenenfalls durch Halogen und/oder durch gegebenenfalls halogen-substituierte Reste aus der Reihe $C_1$-$C_4$-alkyl, $C_1$-$C_4$-Alkoxy, $C_1$-$C_4$-Alkylthio oder $C_1$-$C_2$-Alkylendioxy substituiertes Phenyl steht oder worin die beiden Reste $R^6$ und $R^5$ zusammen für $C_2$-$C_5$-Alkandiyl (Alkylen) stehen ;

in welcher weiter R für den Rest

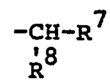

steht, worin

$R^7$ für gegebenenfalls durch halogen und/oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^8$ für Isopropyl oder Cyclopropyl steht ;

$R^1$ für Wasserstoff oder Cyano steht,

X für Chlor oder Brom sthet,

dadurch gekennzeichnet, daß man Carbonsäuren oder deren Salze der Formel II

$$R—CO—OM \qquad (II)$$

in welcher

R die oben angegebene Bedeutung hat und

M für Wasserstoff, Natrium oder Kalium steht, oder reaktionsfähige Derivate derselben

mit 4-Fluor-3-halophenoxy-benzylalkoholen oder reaktionsfähigen Derivaten derselben der Formel III

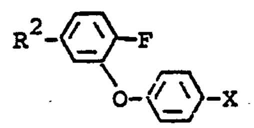

(III)

in welcher

X die oben angegebene Bedeutung hat und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$

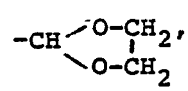

oder

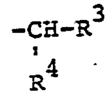

steht,

$R^4$ für die oben bei $R^1$ angegebenen Reste außer Wasserstoff steht und

$R^3$ für OH oder Halogen steht,

gegebenenfalls in Gegenwart von Säureakzeptoren, gegebenenfalls in gegenwart von Alkalicyaniden und gegebenenfalls in Gegenwart von Verdünnungsmitteln umsetzt ;

3. neue 4-Fluor-3-halophenoxy-benzylalkohole und deren reaktionsfähige Derivate der Formel III

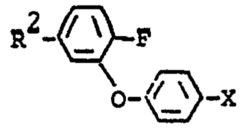

(III)

in welcher

X für Chlor oder Brom steht und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

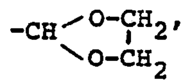

oder

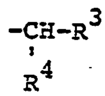

steht,

$R^4$ für CN, $C_{1-3}$-Alkyl, $C_{2-3}$-Alkenyl, $C_{2-3}$-Alkinyl die jeweils durch Halogen substituiert sein können und

$R^3$ für OH oder Halogen steht ;

4. ein Verfahren zur Herstellung von 4-Fluor-3-halogenphenoxybenzylalkoholen und deren reaktionsfähigen Derivaten der Formel III,

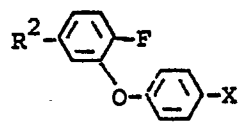

(III)

in welcher

X für Chlor oder Brom steht und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

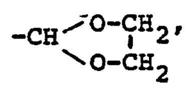

oder

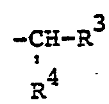

steht.

$R^4$ für CN, $C_{1-3}$-Alkyl, $C_{2-3}$-Alkenyl, $C_{2-3}$-Alkinyl die jeweils durch Halogen substituiert sein können und

$E^3$ für OH oder Halogen steht,

dadurch gekennzeichnet, daß man Verbindungen der Formel IV

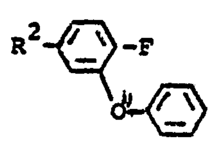

(IV)

in welcher $R^2$ die oben angegebene Bedeutung hat, mit Chlor bzw. Brom gegebenenfalls in Gegenwart eines Katalysators und gegebenenfalls in Gegenwart eines Verdünnungsmittels bei Temperaturen zwischen − 20 und 100 °C umsetzt und gegebenenfalls anschließend nach üblichen Methoden hydrolysiert bzw. reduziert.

Die neuen 4-Fluor-3-halophenoxy-benzylester der Formel (I) zeichnen sich durch hohe pestizide, insbesondere insektizide und akarizide Wirksamkeit aus.

Überraschenderweise zeigen die erfindungsgemäßen Verbindungen der Formel (I) eine erheblich höhere insektizide und akarizide Wirkung als aus dem Stand der Technik bekannte Verbindungen

analoger Struktur und gleicher Wirkungsrichtung und sind zudem wesentlich weniger toxisch gegen Nutztiere, wie z. B. Fische, als ähnliche Verbindungen.

In einer bevorzugten Variante (a) des oben unter (2) dargelegten Herstellungsverfahrens (« Verfahren (2a) ») werden Carbonsäurechloride der Formel IIa

$$R—CO—Cl \qquad (IIa)$$

in welcher R die oben angegebene Bedeutung hat, mit 4-Fluor-3-halophenoxy-benzylalkoholen der Formel (III) (oben) in Gegenwart von Säureakzeptoren und von Verdünnungsmitteln umgesetzt.

In einer weiteren bevorzugten Variante (b) des oben unter (2) dargelegten Herstellungsverfahrens (« Verfahren (2b) » ) — zur Herstellung von Verbindungen der Formel (I), worin $R^1$ für Cyano steht — werden Carbonsäurechloride der Formel IIa (oben) mit 4-Fluor-3-halophenoxy-benzaldehyden der Formel V

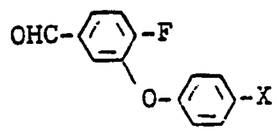

(V)

in welcher X für Chlor oder Brom steht, und wenigstens der äquimolaren Menge eines Alkalicyanids (Natrium- oder Kalium-cyanid) in Gegenwart von Wasser und eines mit Wasser nicht mischbaren organischen Lösungsmittels und gegebenenfalls in Gegenwart eines Katalysators umgesetzt.

Als weitere reaktionsfähige Derivate der Carbonsäure der Formel (II) sind deren Niederalkylester zu nennen, welche mit Alkoholen der Formel (III) nach üblichen Methoden zu Verbindungen der Formel (I) umgesetzt werden können.

Alkali-, Erdalkali- und Ammoniumsalze von Carbonsäuren der Formel (II) können mit Benzylhalogeniden, welche sich von Benzylalkoholen der Formel (III) ableiten, ebenfalls nach üblichen Methoden zu Verbindungen der Formel (I) umgesetzt werden.

Verwendet man als Ausgangsstoffe beispielsweise 3-Methyl-2-phenyl-butansäurechlorid und bei Verfahren (2a) 4-Fluor-3-(4-brom-phenoxy)-benzylalkohol bzw. bei Verfahren (2b) 4-Fluor-3-(4-chlor-phenoxy)-benzaldehyd, so können die bei den beiden Verfahrensvarianten ablaufenden Reaktionen durch folgende Formelschemata skizziert werden :

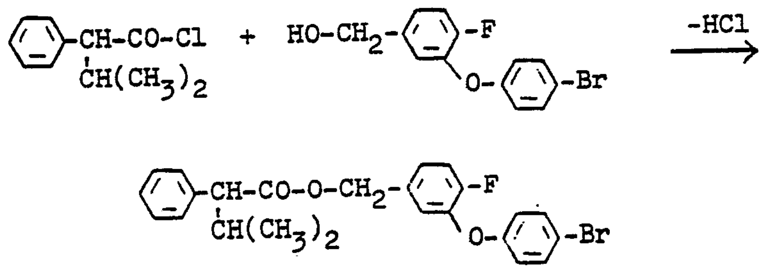


(2a)

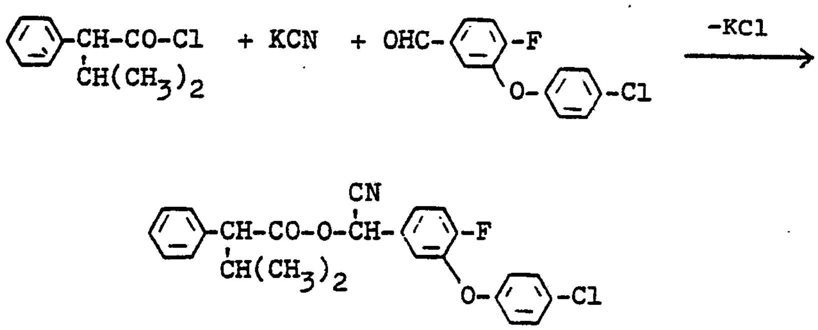


(2b)

Das Verfahren zur Herstellung der neuen Verbindungen der Formel (I) wird in allen Varianten vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl- und Methyl-isobutyl-keton, Ester, wie Essigsäure-methylester und -ethylester, Nitrile, wie z. B. Acetonitril und Propionitril, amide wie z. B. Dimethylformamid, Dimethylacetamid und N-Methylpyrrolidon, sowie Dimethylsulfoxid, Tetramethylensulfon und Hexamethylphosphorsäuretriamid.

Die Variante (a) des erfindungsgemäßen Verfahrens (2) wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliummmethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante (b) des erfindungsgemäßen Verfahrens (2) wird in Gegenwart von Wasser und eines der oben genannten organischen Lösungsmittel, soweit mit Wasser nicht mischbar, durchgeführt. Hierfür sind insbesondere die oben genannten Kohlenwasserstoffe geeignet.

Als Katalysatoren werden bei der Verfahrensvariante (b) vorzugsweise Verbindungen verwendet, welche zum Transfer von Anionen aus Wasser in organische Lösungsmittel geeignet sind. Beispiele hierfür sind Benzyl-triethyl-ammonium-hydrogensulfat, Tetrabutylammonium-bromid und Methyl-trioctyl-ammonium-chlorid (Aliquat 336 ®).

In allen Verfahrensvarianten kann die Reaktionstemperatur innerhalb eines größeren Bereichs variiert werden. Im allgemeinen arbeitet man zwischen 0 und 100 °C, vorzugsweise bei 10 bis 50 °C.

Das erfindungsgemäße Verfahren wird im allgemeinen bei Normaldruck durchgeführt.

Zur Durchführung des erfindungsgemäßen Verfahrens (2) werden die Ausgangsstoffe gewöhnlich in äquimolaren Mengen eingesetzt. Ein Überschuß der einen oder anderen Reaktionskomponente bringt keine wesentlichen Vorteile. Die Ausgangsstoffe werden in Geeigneten Verdünnungsmitteln zusammen gegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z. B. Toluol, verdünnt, die organische Phase abtrennt, mit Wasser wäscht, trocknet, filtriert und vom Filtrat das Lösungsmittel unter vermindertem Druck und bei mäßig erhöhter Temperatur sorgfältig abdestilliert (« Andestillieren »).

Die bei Verfahren (2) als Ausgangsstoffe zu verwendenden Carbonsäuren sind durch Formel (II), die entsprechenden Säurechloride durch Formel (IIa) definiert.

R hat darin vorzugsweise die gleiche Bedeutung, wie sie bei der Definition der Verbindungen der Formel (I) als bevorzugt angegeben ist.

Als Beispiele für die Ausgangsverbindungen der Formel (II) bzw. (IIa) seien genannt :

2,2,3,3-Tetramethyl-cyclopropancarbonsäure und das entsprechende Säurechlorid, 3-(2-Methyl-1-propenyl)-, 3-(2,2-Dichlor-vinyl), 3-(2,2-Difluor-vinyl)-, 3-(2,2-Dibromvinyl)-, 3-(2-Phenyl-vinyl)-, 3-(2-(4-Chlor-phenyl)-vinyl-, 3-(2-Chlor-2-(4-chlor-phenyl)-vinyl)-, 3-(2-Chlor-2-phenyl-vinyl)- und 3-(3,3,3-Trifluor-2-chlor-1-propenyl)-2,2-dimethyl-cyclopropancarbonsäure und die entsprechenden Säurechloride ; Phenyl-, 4-Chlor-phenyl-, 4-Methyl-phenyl-, 4-tert-Butyl-phenyl-, 4-Trifluormethyl-phenyl-, 4-Methoxy-phenyl-, 4-Trifluormethoxy-phenyl- und 3,4-Methylendioxy-phenyl-X-isopropylessigsäure und die entsprechenden Säurechloride.

Die Carbonsäuren der Formel (II) sowie entsprechende Säurechloride der Formel (IIa) sind bekannt (vergleiche US-PS 3 996 244, 4 157 447, 3 962 458, 3 835 176 ; GB-PS 1 413 491 und 2 000 764).

Die weiter als Ausgangsstoffe zu verwendenden 4-Fluor-3-halophenoxy-benzylalkohole bzw. ihre reaktionsfähigen Derivate sind durch die Formeln (III) definiert. Vorzugsweise stehen darin

X für Chlor oder Brom,

$R^2$ für den Rest —CHO, oder $-\overset{R^4}{\overset{|}{C}}H-R^3$ wobei

$R^4$ für Wasserstoff oder Cyano steht

$R^3$ für Hydroxy steht.

Als Beispiele für die Verbindungen der Formel (III) seien genannt :

4-Fluor-3-(4-chlor-phenoxy)-benzylalkohol, 4-Fluor-3-(4-brom-phenoxy)-benzylalkohol, 4-Fluor-3-(4-chlorphenoxy)-benzaldehyd und 4-fluor-3-(4-brom-phenoxy)-benzaldehyd.

Man erhält die neuen 4-Fluor-3-halophenoxy-benzylalkohole (III) bzw. deren reaktionsfähige Derivate, wie oben unter (4) dargelegt, wenn man Verbindungen der Formel IV (oben), wie z. B. 4-Fluor-3-phenoxy-benzaldehyd, mit Chlor- bzw. Brom oder Chlorbrom, gegebenenfalls in Gegenwart eines Verdünnungsmittels, wie z. B. Essigsäure, bei Temperaturen zwischen – 20 und 100 °C, gegebenenfalls in Gegenwart eines Katalysators, wie z. B. Jod, Schwefel, Dischwefeldichlorid, Eisen oder Eisen (III) chlorid, vorzugsweise bei 10 bis 60 °C, umsetzt und gegebenenfalls anschließend nach üblichen Methoden Umwandlungen in andere Verbindungen der Formel (III) durchführt. Die Aufarbeitung kann nach üblichen Methoden durchgeführt werden, beispielsweise, indem man mit Wasser verdünnt und das sich abscheidende Produkt durch Abdekantieren oder Absaugen isoliert. Die so erhaltenen Rohprodukte können nach üblichen Methoden gereinigt werden.

Ausgangsverbindungen der Formel IV sind bereits bekannt (vergleiche DE-OS 2 709 264).

Die Verbindungen der Formel I eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor

vorkommen. Sie sind gegen normal sensible und resistance Arten sowie gegen alle oder einzelne Entwicklungsstadien wirksam. Zu den oben erwähnten Schädlingen gehören :

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung des Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus heredera, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earis insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotacsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermetes spp., Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Ambyomma spp., Hyalomma spp., Ixodes spp., psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie

Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit Verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfage, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirsktoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,000 1 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirsktoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (dippen), Sprühens (Sprayen), Aufgießens (pour-on and spont-on) und des Einpuders sowie durch parenterale Anwendung in Form beispielsweise der Injektion.

## Herstellungsbeispiele

### Beispiel 1

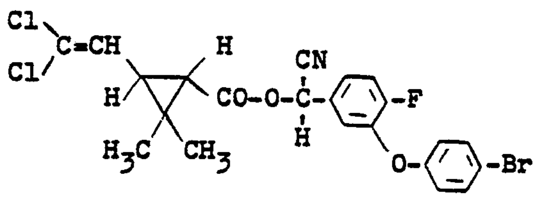

Eine Mischung von 14,75 g (0,05 Mol) 3-(4-Brom-phenoxy)-4-fluorbenzaldehyd und 11,37 g (0,05 Mol) (±)-trans-2,2-Dimethyl-3-(2,2-dichlor-vinyl)-cyclopropancarbonsäurechlorid, gelöst in 40 ml Cyclohexan, werden unter Rühren bei 20-25 °C zu einer Mischung von 3,9 g Natriumcyanid, 5,8 ml Wasser, 300 ml Cyclohexan und 0,9 Tetrabutylammoniumbromid getropft und dann 4 Stunden bei 20-25 °C gerührt.

Anschließend wird die Reaktionsmischung mit 400 ml Toluol versetzt und zweimal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60 °C/1 Torr Badtemperatur entfernt. Man erhält 21,7 g (84,6 % der Theorie (±)-trans-2,2-Dimethyl-3-(2,2-dichlor-vinyl)-cyclopropancarbonsäure-(±)-X-cyano-3-(4-brom-phenoxy)-4-fluor-benzylester als zähes Öl.

$^1$H-NMR-Spektrum ($CDCl_3$/TMS), τ (ppm)

Dimethyl-H : 8,6-8,83 (m/6 H) ; Cyclopropan-H : 8,26-8,43 (m/1 H) und 7,6-7,82 (m/1 H) ; Vinyl-H : 4,38 (d/1 H) ; Benzyl-H : 3,64 (S/1 H) ; aromat.-H : 2,4-3,2 (m/7 H).

Analog Beispiel 1 können folgende Verbindungen hergestellt werden :

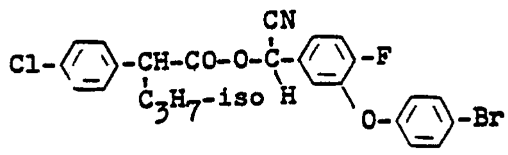

Ausbeute : 87,3 % der Theorie

(2)

$^1$H-NMR-Spektrum ($CDCL_3$/TMS, τ (ppm)
Benzyl-H : 3,69 (S/$^1$/2 H) und 3,73 (S/$^1$/2 H).

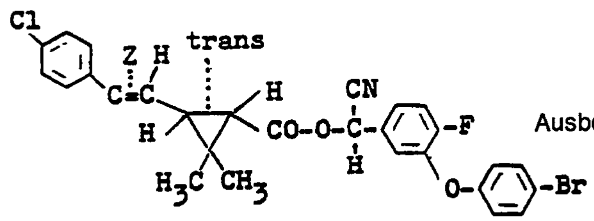

Ausbeute : 85,2 % der Theorie

(3)

Benzyl-H : 3,62 (S/1 H)

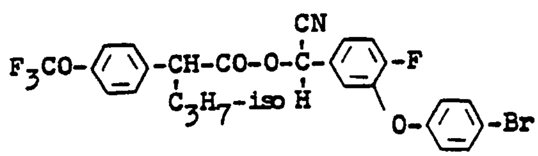

Ausbeute : 79,8 % der Theorie

(4)

Benzyl-H : 3,69 (S/$^1$/2 H) und 3,73 (S/$^1$/2 H)

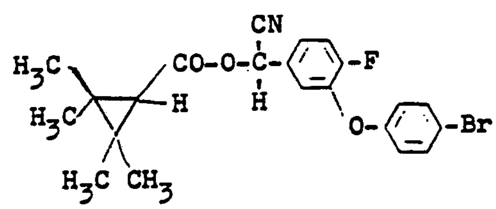

Ausbeute : 77,6 % der Theorie

(5)

Benzyl-H : 3,60 (S/1 H) ; Methyl-H : 8,69-8,85 (m/12 H)

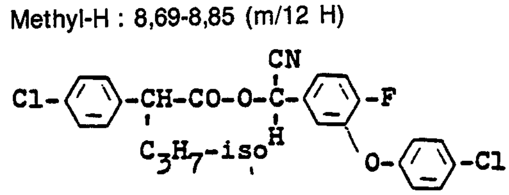

Ausbeute : 67,5 % der Theorie

(6)

Benzyl-H : 3,69 (S/$^1$/2 H) und 3,73 (S/ $^1$/2 H)

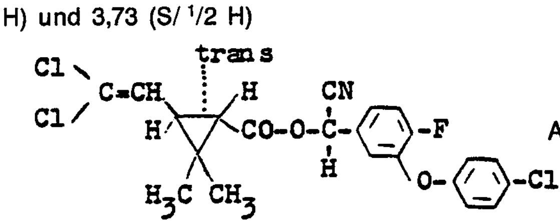

Ausbeute : 82,6 % der Theorie

(7)

Benzyl-H : 3,64 (S/ $^1$/2 H) und 3,66 (S/ $^1$/2 H) ;
Vinyl-H : 4,38 (d/ $^1$/2 H) und 4,41 (d/ $^1$/2 H)

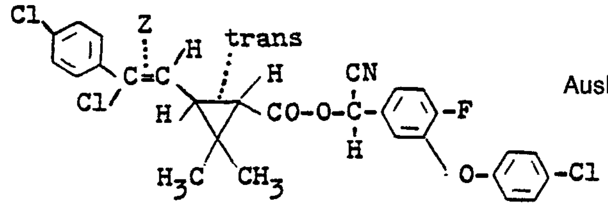

Ausbeute : 83 % der Theorie

(8)

Benzyl-H : 3,62 (S/ 1/2 H) und 3,64 (S/ 1/2 H) ;
Vinyl-H : 4,17 (d/1 H)

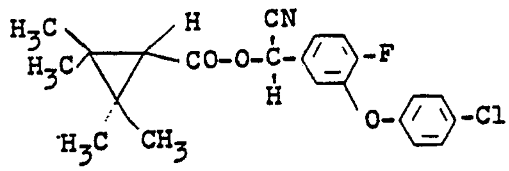

Ausbeute : 78 % der Theorie

(9)

Benzyl-H : 3,67 (S/1 H) ; Methyl-H : 8,68-8,85 (m/12 H)

Beispiel 2

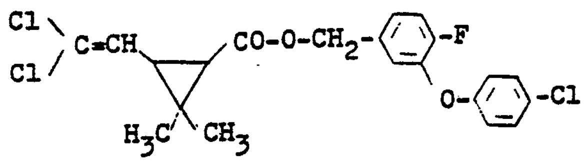

(10)

5,5 g (0,022 Mol) 3-(4-Chlor-phenoxy)-4-fluor-benzylalkohol und 4,96 g (0,022 Mol (±)-trans-3-(2,2-Dichlor-vinyl)-2,2-dimethyl-cyclopropan-carbonsäurechlorid werden in 100 ml wasserfreiem Toluol gelöst und bei 20-25 °C 2,5 g Pyridin, gelöst in 50 ml wasserfreiem Toluol, unter Rühren zugetropft. Anschließend wird weitere 3 Stunden bei 25 °C gerührt. Das Reaktionsgemisch wird in 150 ml Wasser, dem 10 ml konz. Salzsäure zugesetzt werde, gegossen, die organische Phase abgetrennt und nochmals mit 100 ml Wasser gewaschen. Anschließend wird die Toluolphase über Natriumsulfat getrocknet und das Lösungsmittel in Wasserstrahlvakuum abdestilliert. Letzte Lösungsmittelreste werden durch kurzes Andestillieren bei 60 °C/1 Torr Badtemperatur entfernt. Man erhält 7,9 g (81 % der Theorie) 3-(4-Chlorphenoxy)-4-fluorbenzyl-(±)-trans-3-(2,2-dichlorvinyl)-2,2-dimethyl-cyclopropancarbonsäureester als gelbes Öl. Die Struktur wird durch das $^1$H-NMR-Spektrum bewiesen.

$^1$H-NMR-Spektrum in $CDCl_3$/τ (ppm) :
Benzyl-H : 4,97 (S/2 H)
Analog Beispiel 2 erhält man :

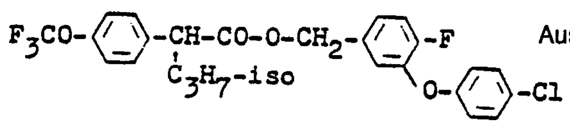

Ausbeute : 72,8 % der Theorie

(11)

$^1$H-NMR-Spektrum $CDCl_3$/τ (ppm) :
Benzyl-H : 4,98 (S/2 H)

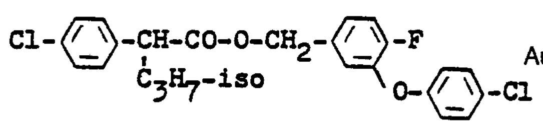

Ausbeute : 85,6 % der Theorie

Benzyl-H : 4,97 (S/2 H)
Beispiele zur Herstellung der Ausgangsverbindungen :
a) Herstellung von 4-Fluor-3-(4-bromphenoxy)-benzaldehyd

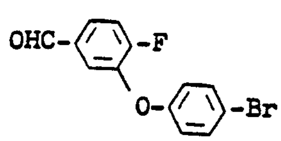

136,4 g (0,6 Mol : 95 %ig) 3-Phenoxy-4-fluorbenzaldehyd werden zusammen mit 130 g Eisessig vorgelegt. Unter Rühren wird eine Mischung aus 96 g (0,6 Mol) brom und 30 g Eisessig bei 50 °C so zugetropft, daß, sich die Temperatur von selbst hält. Anschließend wird 30 Minuten nachgerührt. Die orangerote Reaktionslösung wird in 1 l kaltes Wasser gerührt. Das sich als Öl abscheidende Reaktionsprodukt erstarrt bald zu einer festen Kristallmasse. Danach wird kalt abgesaugt und durch Wasserwäsche noch anhaftendes Brom entfernt. Ausbeute = 169 g (lufttrocken).

Das Rohprodukt wird in 800 ml Hexan, dem wenig Äthanol zugesetzt ist, bei 40-45 °C gelöst, auf 0 °C abgekühlt, die abgeschiedenen Kristalle abgesaugt und mit etwas Hexan gewaschen. Ausbeute = 124 g = 70 % der Theorie ; Schmelzpunkt : 79-80 °C.

b) Herstellung von 4-Fluor-3-(4-chlorphenoxy)-benzaldehyd

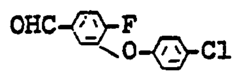

227,5 g (1 Mol ; 95 %ig) 3-Phenoxy-4-fluor-benzaldehyd wird zusammen mit 250 g Eisessig bei Raumtemperatur vorgelegt und innerhalb 1 Stunde 71 g (1 Mol) Chlor eingeleitet. Durch leichte Außenkühlung wird die Temperatur bei 35 °C gehalten. Anschließend wird 30 Minuten nachgerührt. Die leicht gelbgefärbte Reaktionslösung wird in ca. 1,5 l kaltes Wasser gerührt. Bald entsteht eine weiße Kristallschmiere. Diese wird einige Male mit Wasser verrührt und abdekantiert, anschließend mit 260 g = 300 ml Toluol aufgenommen, mit verdünnter Sodalösung ausgeschüttelt, 3 × mit je 100 ml Hydrochlorid ausgewaschen und bei max. 90 und 30 mbar am Rotationsverdampfer eingeengt. Rückstand 275 g gelbes Öl.

Reinigung : 275 g Öl wurden bei 40 °C in 200 ml Cyclohexan-Ethanol-Gemisch (9 : 1 Vol. Te) aufgenommen, auf 0 °C abgekühlt, die abgeschiedenen Kristalle abgesaugt und mit ca. 300 ml Cyclohexan-Ethanol-Gemisch kalt nachgewaschen. Ausbeute : 88 g = 35 % der Theorie (weiße Kristalle) vom Schmelzpunkt 66,5-68 °C.

c) Herstellung von 4-Fluor-3-(4-chlorphenoxy)-benzylalkohol

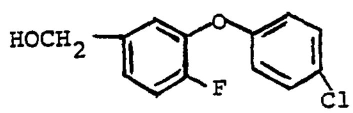

Zu einer Mischung von 1,73 g Natriumborhydrid, 25 ml Dioxan und 3 ml Wasser werden bei 10 °C unter Rühren 7,5 g (0,03 Mol) 3-(4-Chlor-phenoxy)-4-fluorbenzaldehyd, gelöst in 20 ml Dioxan, zugetropft und die Mischung anschließend 10 Stunden bei Raumtemperatur gerührt. Anschließend wird die Reaktionsmischung in 150 ml Wasser gegossen und zweimal mit 100 ml Methylenchlorid extrahiert. Die vereinigten Methylenchloridphasen werden über Magnesiumsulfat getrocknet und anschließend das Lösungsmittel im Vakuum abdestilliert. Man erhält 6,2 g (82 % der Theorie) 3-(4-Chlor-phenoxy)-4-fluorbenzylalkohol als gelbliches Öl das nach kurzer Zeit kristallisiert, Schmelzpunkt 65-67 °C.

In den folgenden Beispielen A-G werden als Verbindungen zum Stand der Technik Permethrin und Cypermethrin (GB-PS 1 413 491) eingesetzt.

## Beispiel A

Drosophila-Test

Lösungsmittel : 3 Gewichtsteile Aceton

Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese naß auf die Öffnung eines Glasgefäßes, in dem sich 50 Taufliegen (Drosophila melanogaster) befinden und bedeckt es mit einer Glasplatte.

Nach der gewünschten Zeit bestimmt man die Abtötung in %. Dabei bedeutet 100 %, daß alle Fliegen abgetötet wurden ; 0 % bedeutet, daß keine Fliegen abgetötet wurden.

Bei diesem Test ziegen z. B. die folgenden Verbindungen der Herstellungsbeispiele überlegene Wirksamkeit gegenüber dem Stand der Technik : 1, 3, 8, 9, 7, 6, 2.

## Beispiel B

Tetranychus-Test (resistent)

Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden ; 0 % bedeutet, daß keine Spinnmilben abgetötet wurden.

Bei diesem Test zeigen z. B. die folgenden Verdindungen der Herstellungsbeispiele überlegene Wirksamkeit gegenüber dem Stand der Technik : 9, 4, 5.

Beispiel C

Grenzkonzentrations-Test/Bodeninsekten

Testinsekt : Tenebrio Molitor-Larven (im Boden)
Lösungsmittel : 3 Gewichtsteile Aceton
Emulgator : 1 Gewichtsteil Alkylarylpolyglykoläther

Zur Herstellung einer zweckmäßigen Wirsktoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik : 2, 3, 4, 7.

Beispiel D

Grenzkonzentrations-Test/Bodeninsekten

Testinsekt : Agrotis segetum-Larven (im Boden)
Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik : 3.

Beispiel E

$LD_{100}$-Test

Testtiere : Blatta orientalis
Zahl der Testtiere : 10
Lösungsmittel : Aceton

2 Gewichtsteile Wirkstoff werden in 1 000 Volumenteilen Lösungsmittel aufgenommen. Die so erhaltene Lösung wird mit weiterem Lösungsmittel auf die gewünschten Konzentration verdünnt.

2,5 ml Wirkstofflösung werden in eine Petrischale pipettiert. Auf dem Boden der Petrischale befindet

sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden ; 0 % bedeutet, daß keine Testtiere abgetötet wurden.

Bei diesem Test zeigen z. B. die folgenden Verbindungen der Herstellungsbeispiele überlegene Wirkung gegenüber dem Stand der Technik : 1, 7, 5.

Beispiel F

Test mit Boophilius microplus resistent

Lösungsmittel : 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilius microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik : 1, 2, 3,4, 5, 6, 7, 8, 9.

Beispiel G

Test mit Lucilia cuprina res.-Larven

Emulgator : 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik : 1, 2, 3, 4, 5, 8, 9.

**Ansprüche**

1. 4-Fluor-3-halophenoxy-Benzylester der Formel (I)

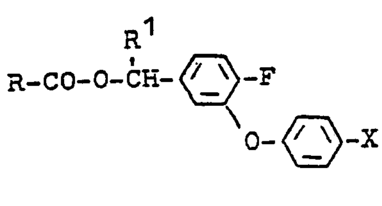

(I)

in welcher R für 2,2,3,3-Tetramethylcyclopropyl oder für den Rest

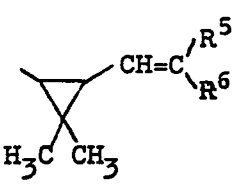

steht, worin

$R^5$ für Wasserstoff, Fluor, Chlor, Brom oder für gegebenenfalls halogen-substituiertes $C_1$-$C_4$-Alkyl steht und

$R^6$ für Fluor, Chlor, Brom, gegebenenfalls halogen-substituiertes $C_1$-$C_4$-Alkyl oder für gegebenenfalls durch Halogen und/oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht oder worin die beiden Reste $R^6$ und $R^5$ zusammen für $C_2$-$C_5$-Alkandiyl (Alkylen) stehen ;

in welcher weiter R für den Rest

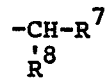

steht, worin

$R^7$ für gegebenenfalls durch Halogen und/oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^8$ für Isopropyl oder Cyclopropyl steht ;

$R^1$ für Wasserstoff oder Cyano steht,

X für Chlor oder Brom steht.

2. Verfahren zur Herstellung der 4-Fluor-3-halophenoxybenzylester der Formel I

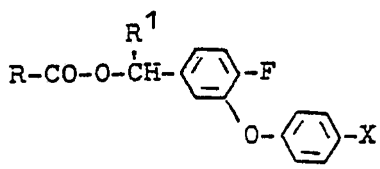

(I)

in welcher R für 2,2,3,3-Tetramethylcyclopropyl oder für den Rest

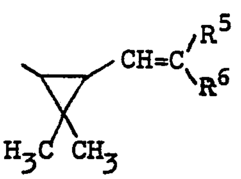

steht, worin

$R^5$ für Wasserstoff, Fluor, Chlor, Brom oder für gegebenenfalls halogen-substituiertes $C_1$-$C_4$-Alkyl steht und

$R^6$ für Fluor, Chlor, Brom, gegebenenfalls halogen-substituiertes $C_1$-$C_4$-Alkyl oder für gegebenenfalls durch Halogen und/oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht oder worin die beiden Reste $R^6$ und $R^5$ zusammen für $C_2$-$C_5$-Alkandiyl (Alkylen) stehen ;

in welcher weiter R für den Rest

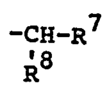

steht, worin

$R^7$ für gegebenenfalls durch Halogen und/oder durch gegebenenflalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^8$ für Isopropyl oder Cyclopropyl steht ;

$R^1$ für Wasserstoff oder Cyano steht,

X für Chlor oder Brom steht,

dadurch gekennzeichnet, daß man Carbonsäuren oder deren Salze der Formel II

$$R—CO—OM \qquad (II)$$

in welcher

R die oben angebene Bedeutung hat und

M für Wasserstoff, Natrium oder Kalium steht, oder reaktionsfähige Derivate derselben

mit 4-Fluor-3-halophenoxy-benzylalkoholen oder reaktionsfähigen Derivaten derselben der Formel III

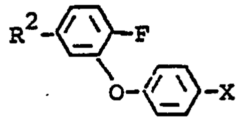

(III)

in welcher

X die oben angegebene Bedeutung hat und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

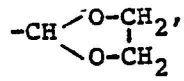

oder

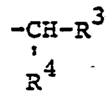

steht,

$R^4$ für die oben bei $R^1$ angegebenen Reste außer Wasserstoff steht und

$R^3$ für OH oder Halogen steht,

gegebenenfalls in Gegenwart von Säureakzeptoren, gegebenenfalls in Gegenwart von Alkalicyaniden und gegebenenfalls in Gegenwart von Verdünnungsmitteln umsetzt.

3. 4-Fluor-3-halophenoxy-benzylalkohole und deren reaktionsfähige Derivate der Formel III

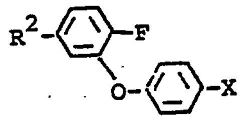

(III)

in welcher

X für Chlor oder Brom steht und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

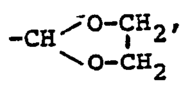

oder

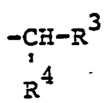

steht,

$R^4$ für CN, $C_{1-3}$-Alkyl, $C_{2-3}$-Alkenyl, $C_{2-3}$-Alkinyl die jeweils durch Halogen substituiert sein können und

$R^3$ für OH oder Halogen steht.

4. Verfahren zur Herstellung von 4-Fluor-3-halogenphenoxybenzylalkoholen und deren reaktionsfähigen Derivaten der Formel III,

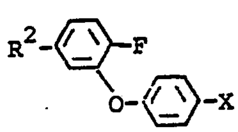

(III)

in welcher

X für Chlor oder Brom steht und

$R^2$ für $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

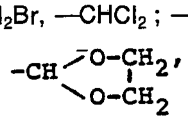

oder

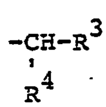

steht.

$R^4$ für CN, $C_{1-3}$-Alkyl, $C_{2-3}$-Alkenyl, $C_{2-3}$-Alkinyl die jeweils durch Halogen substituiert sein können und

$R^3$ für OH oder Halogen steht,

dadurch gekennzeichnet, daß man Verbindungen der Formel IV

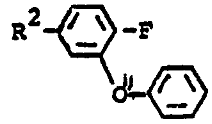

(IV)

in welcher $R^2$ die oben angegebene Bedeutung hat, mit Chlor bzw. Brom gegebenenfalls in Gegenwart eines Katalysators und gegebenenfalls in Gegenwart eines Verdünnungsmittels bei Temperaturen zwischen − 20 und 100 °C umsetzt und gegebenenfalls anschließend nach üblichen Methoden hydrolysiert bzw. reduziert.

5. Schädlingsbekämpfungsmittel, gekennzeichnet durch einen Gehalt an mindestens einem 4-Fluor-3-halo-phenoxy-benzylester der Formel (I).

6. Verwendung von 4-Fluor-3-halophenoxy-benylester der Formel (I) zur Bekämpfung von Schädlingen.

7. Verfahren zur Bekämpfung von Schädlingen, dadurch gekennzeichnet, daß man 4-Fluor-3-halophenoxy-benzylester der Formel (I) auf Schädlinge und/oder ihren Lebensraum einwirken läßt.

8. Verfahren zur Herstellung von Schädlingsbekämpfungsmitteln, dadurch gekennzeichnet, daß man 4-Fluor-3-halophenoxy-benzylester der Formel (I) mit Streckmitteln und/oder oberflächenaktiven Mitteln vermischt.

**Claims**

1. 4-Fluoro-3-halophenoxybenzyl esters of the formula (I)

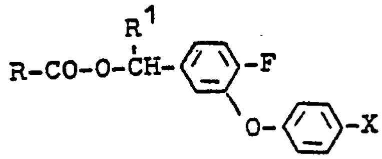

(I)

in which R represents 2,2,3,3-tetramethylcyclopropyl or the radical

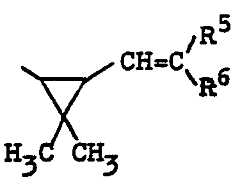

wherein

$R^5$ represents hydrogen, fluorine, chlorine, bromine or optionally halogen-substituted $C_1$-$C_4$-alkyl and

$R^6$ represents fluorine, chlorine, bromine, optionally halogen-substituted $C_1$-$C_4$-alkyl or phenyl which is optionally substituted by halogen and/or by optionally halogen-substituted radicals from the series comprising $C_1$-$C_4$-alkyl, $C_1$-$C_4$-alkoxy, $C_1$-$C_4$-alkylthio or $C_1$-$C_2$-alkylenedioxy, or wherein the two radicals $R^6$ and $R^5$ together represent $C_2$-$C_5$-alkanediyl (alkylene) ;

in which, further, R represents the radical

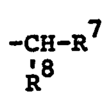

wherein

$R^7$ represents phenyl which is optionally substituted by halogen and/or by optionally halogen-substituted radicals from the series comprising $C_1$-$C_4$-alkyl, $C_1$-$C_4$-alkoxy, $C_1$-$C_4$-alkylthio or $C_1$-$C_2$-alkylenedioxy and

$R^8$ represents isopropyl or cyclopropyl ;

$R^1$ represents hydrogen or cyano and

X represents chlorine or bromine.

2. Process for the preparation of the 4-fluoro-3-halophenoxybenzyl esters of the formula I

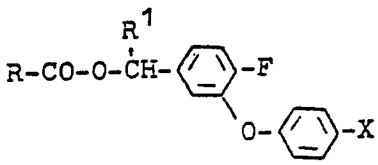

(I)

in which R represents 2,2,3,3-tetramethylcyclopropyl or the radical

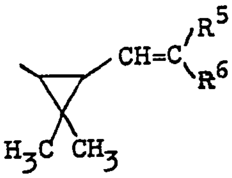

wherein

$R^5$ represents hydrogen, fluorine, chlorine, bromine or optionally halogen-substituted $C_1$-$C_4$-alkyl and

$R^6$ represents fluorine, chlorine, bromine, optionally halogen-substituted $C_1$-$C_4$-alkyl or phenyl which is optionally substituted by halogen and/or by optionally halogen-substituted radicals from the series comprising $C_1$-$C_4$-alkyl, $C_1$-$C_4$-alkoxy, $C_1$-$C_4$-alkylthio or $C_1$-$C_2$-alkylenedioxy, or wherein the two radicals $R^6$ and $R^5$ together represent $C_2$-$C_5$-alkanediyl (alkylene) ;

in which, further, R represents the radical

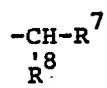

wherein

$R^7$ represents phenyl which is optionally substituted by halogen and/or by optionally halogen-substituted radicals from the series comprising $C_1$-$C_4$-alkyl, $C_1$-$C_4$-alkoxy, $C_1$-$C_4$-alkylthio or $C_1$-$C_2$-alkylenedioxy and

$R^8$ represents isopropyl or cyclopropyl ;

$R^1$ represents hydrogen or cyano and

X represents chlorine or bromine,

characterised in that carboxylic acids, or salts thereof, of the formula II

$$R—CO—OM \qquad (II)$$

in which

R has the abovementioned meaning and

M represents hydrogen, sodium or potassium,

or reactive derivatives thereof, are reacted with 4-fluoro-3-halophenoxybenzyl alcohols, or reactive derivatives thereof, of the formula III

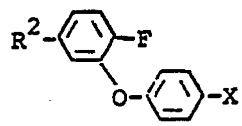

(III)

in which

X has the abovementioned meaning and

$R^2$ represents —CHO, —$CH_2OH$, —$CH_2Cl$, —$CH_2Br$, —$CHCl_2$ ; —$CHBr_2$ ; —$CH(OCH_3)_2$, —$CH(OC_2H_5)_2$,

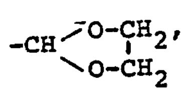

or

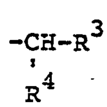

$R^4$ represents the radicals given above in the case of $R^1$, except for hydrogen, and
$R^3$ represents OH or halogen,
if appropriate in the presence of acid acceptors, if appropriate in the presence of alkali metal cyanides and if appropriate in the presence of diluents.

3. 4-Fluoro-3-halophenoxybenzyl alcohols, and reactive derivatives thereof, of the formula III

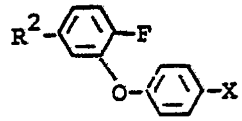

(III)

in which
X represents chlorine or bromine and
$R^2$ represents —CHO, —$CH_2OH$, —$CH_2Cl$, —$CH_2Br$, —$CHCl_2$; —$CHBr_2$; —$CH(OCH_3)_2$, —$CH(OC_2H_5)_2$,

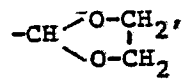

or

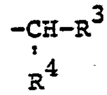

$R^4$ represents CN, $C_{1-3}$-alkyl, $C_{2-3}$-alkenyl, or $C_{2-3}$-alkinyl, each of which can be substituted by halogen and
$R^3$ represents OH or halogen.

4. Process for the preparation of 4-fluoro-3-halogeno-phenoxybenzyl alcohols, and reactive derivatives thereof, of the formula III

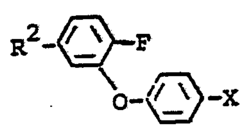

(III)

in which
X represents chlorine or bromine and
$R^2$ represents —CHO, —$CH_2OH$, —$CH_2Cl$, —$CH_2Br$, —$CHCl_2$; —$CHBr_2$; —$CH(OCH_3)_2$, —$CH(OC_2H_5)_2$,

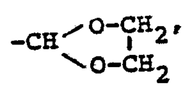

or

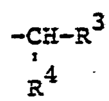

$R^4$ represents CN, $C_{1-3}$-alkyl, $C_{2-3}$-alkenyl, or $C_{2-3}$-alkinyl, each of which can be substituted by halogen and
$R^3$ represents OH or halogen,
characterised in that compounds of the formula IV

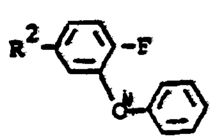

(IV)

in which $R^2$ has the abovementioned meaning,
are reacted with chlorine or bromine, if appropriate in the presence of a catalyst and if appropriate in the presence of a diluent, at temperature between − 20 and 100 °C and, if appropriate, the reaction products are then hydrolysed or reduced according to customary methods.

5. Pest-combating agents, characterised in that they contain at least one 4-fluoro-3-halophenoxybenzyl ester of the formula (I).

6. Use of 4-fluoro-3-halophenoxybenzyl esters of the formula (I) for combating pests.

7. Process for combating pests, characterised in that 4-fluoro-3-halophenoxybenzyl esters of the formula (I) are allowed to act on pests and/or their habitat.

8. Process for the preparation of pest-combating agents, characterised in that 4-fluoro-3-halophenoxybenzyl esters of the formula (I) are mixed with extenders and/or surface active agents.

**Revendications**

1. Esters 4-fluoro-3-halogénophénoxy-benzyliques de formule (I)

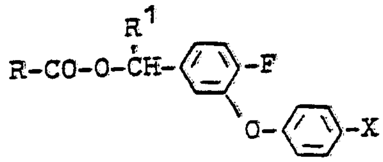

(I)

dans laquelle R est un reste 2,2,3,3-tétraméthylcyclopropyle ou le reste de formule

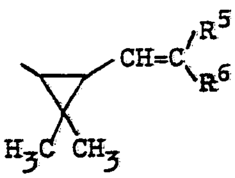

dans laquelle

$R^5$ désigne l'hydrogène, le fluor, le chlore, le brome ou un groupe alkyle en $C_1$ à $C_4$ éventuellement substitué par un halogène et

$R^6$ désigne le fluor, le chlore, le brome, un groupe alkyle en $C_1$ à $C_4$ éventuellement substitué par un halogène ou un groupe phényle éventuellement substitué par un halogène et/ou par des restes de la série alkyle en $C_1$ à $C_4$, alkoxy en $C_1$ à $C_4$, alkylthio en $C_1$ à $C_4$ ou alkylènedioxy en $C_1$ ou $C_2$ éventuellement substitués par des halogènes, ou bien les deux restes $R^6$ et $R^5$ forment conjointement un groupe alcanediyle (alkylène) en $C_2$ à $C_5$ ;

dans laquelle en outre R représente le reste

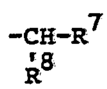

où

$R^7$ désigne un groupe phényle éventuellement substitué par un halogène et/ou par des restes de la série alkyle en $C_1$ à $C_4$, alkoxy en $C_1$ à $C_4$, alkylthio en $C_1$ à $C_4$ ou alkylènedioxy en $C_1$ ou $C_2$ éventuellement substitués par des halogènes et

$R^8$ est un groupe isopropyle ou cyclopropyle ;

$R^1$ désigne l'hydrogène ou le groupe cyano,

X est le chlore ou le brome.

2. Procédé de production des esters 4-fluoro-3-halogénophénoxy-benzyliques de formule I

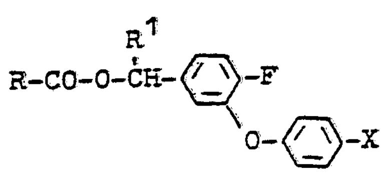

(I)

dans laquelle R est un reste 2,2,3,3-tétraméthylcyclopropyle ou le reste de formule

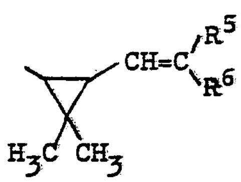

dans laquelle

$R^5$ désigne l'hydrogène, le fluor, le chlore, le brome ou un groupe alkyle en $C_1$ à $C_4$ éventuellement substitué par un halogène et

$R^6$ désigne le fluor, le chlore, le brome, un groupe alkyle en $C_1$ à $C_4$ éventuellement substitué par un halogène ou un groupe phényle éventuellement substitué par un halogène et/ou par des restes de la série alkyle en $C_1$ à $C_4$, alkoxy en $C_1$ à $C_4$, alkylthio en $C_1$ à $C_4$ ou alkylènedioxy en $C_1$ ou $C_2$ éventuellement substitués par des halogènes, ou bien les deux restes $R^6$ et $R^5$ forment conjointement un groupe alcanediyle (alkylène) en $C_2$ à $C_5$ ;

dans laquelle en outre

R représente le reste

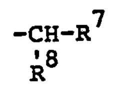

où

$R^7$ désigne un groupe phényle éventuellement substitué par un halogène et/ou par des restes de la série alkyle en $C_1$ à $C_4$, alkoxy en $C_1$ à $C_4$, alkylthio en $C_1$ à $C_4$ ou alkylènedioxy en $C_1$ ou $C_2$ éventuellement substitués par des halogènes et

$R^8$ est un groupe isopropyle ou cyclopropyle ;

$R^1$ désigne l'hydrogène ou le groupe cyano,

X est le chlore ou le brome,

caractérisé en ce qu'on fait réagir des acides carboxyliques ou leurs sels de formule II

$$R—CO—OM \qquad (II)$$

dans laquelle

R a la définition indiquée ci-dessus et

M désigne l'hydrogène, le sodium ou le potassium, ou leurs dérivés réactifs,

avec des alcools 4-fluoro-3-halogénophénoxy-benzyliques ou leurs dérivés réactifs de formule III

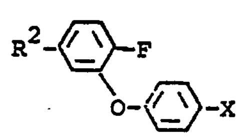

(III)

dans laquelle

X a la définition indiquée ci-dessus et

$R^2$ désigne $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

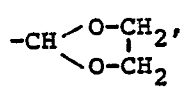

ou

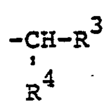

$R^4$ désigne les restes indiqués ci-dessus pour $R^1$, hormis l'hydrogène et

$R^3$ est un groupe OH ou un halogène,

éventuellement en présence d'accepteurs d'acides, en la présence éventuelle de cyanures alcalins et, le cas échéant, en présence de diluants.

3. Alcools 4-fluoro-3-halogénophénoxy-benzyliques et leurs dérivés réactifs de formule III

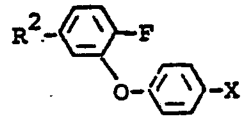

dans laquelle

X est le chlore ou le brome et

$R^2$ représente $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

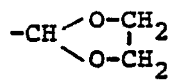

ou

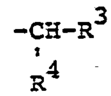

$R^4$ représente un groupe CN, alkyle en $C_1$ à $C_3$, alcényle en $C_2$ ou $C_3$, alcynyle en $C_2$ ou $C_3$ qui peuvent être substitués chacun par un halogène et

$R^3$ représente OH ou un halogène.

4. Procédé de production d'alcools 4-fluoro-3-halogénophénoxy-benzyliques et de leurs dérivés réactifs de formule III

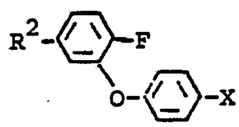

(III)

dans laquelle

X désigne le chlore ou le brome et

$R^2$ représente $—CHO$, $—CH_2OH$, $—CH_2Cl$, $—CH_2Br$, $—CHCl_2$ ; $—CHBr_2$ ; $—CH(OCH_3)_2$, $—CH(OC_2H_5)_2$,

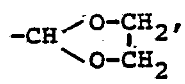

ou

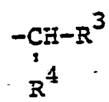

$R^4$ est un groupe CN, alkyle en $C_1$ à $C_3$, alcényle en $C_2$ ou $C_3$, alcynyle en $C_2$ ou $C_3$, chacun pouvant être substitué par un halogène et

$R^3$ représente OH ou un halogène,

caractérisé en ce qu'on fait réagir des composés de formule IV

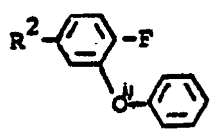

(IV)

dans laquelle $R^2$ a la définition indiquée ci-dessus, avec du chlore ou du brome en présence éventuelle d'un catalyseur et, le cas échéant, en présence d'un diluant, à des températures comprises entre – 20 et 100 °C, puis on effectue éventuellement une hydrolyse ou une réduction par des procédés classiques.

5. Composition pesticide, caractérisée par une teneur en au moins un ester 4-fluoro-3-halogénophénoxy-benzylique de formule (I).

6. Utilisation d'esters 4-fluoro-3-halogénophénoxy-benzyliques de formule (I) pour combattre des parasites.

7. Procédé pour combattre des parasites, caractérisé en ce qu'on fait agir des esters 4-fluoro-3-halogénophénoxy-benzyliques de formule (I) sur les parasites et/ou sur leur habitat.

8. Procédé de préparation de compositions pesticides, caractérisé en ce qu'on mélange des esters 4-fluoro-3-halogénophénoxy-benzyliques de formule (I) avec des diluants et/ou des agents tensio-actifs.